# EUROPEAN PATENT APPLICATION

(11) **EP 2 479 339 A1**
(43) Date of publication of application: **25.07.2012**
(21) Application number: 12151885.6
(22) Date of filing: 20.01.2012
(51) Int. Cl.: D06M 11/42, D06M 11/74, D06M 11/76, A61N 5/06, D01F 6/90, D01F 8/06, D01F 8/12, D02G 3/16, D06M 101/20, D06M 101/34

(54) **Textile yarn radiating far rays and manufacturing methods thereof**

(30) Priority: 20.01.2011 IT TV20110004
(71) Applicant: Idee per il Tessile S.r.l., 31050 Villorba (Treviso) (IT); C.R.T. Studio SNC di Pini G. & Cavaion M., 31044 Montebelluna (Treviso) (IT)
(72) Inventor: Cavaion, Marino, 31044 Montebelluna (Treviso) (IT); Lauretti, Ezio, 31100 Treviso (IT); Lauretti, Pierpaolo, 31050 Ponzano Veneto (Treviso) (IT); Pini, Gabriele, 31010 Farra di Soligo (Treviso) (IT)
(74) Representative: Dragotti, Gianfranco

(57) **Abstract**

The present invention relates to a yarn, for the production of fabrics, suitable for absorbing far infrared rays and subsequently irradiating said rays in the form of FAR rays, said yarn being characterized in that it is composed of:
- Polypropylene (PP) in an amount of between 60% and 80%
- Silver oxide (Ag2O) in an amount of between 0.5 % and 5%.
- Carbon (C60sp2 or Csp2) in an amount of between 5% and 10%
- Polyamide (PA) in amount of between 10% and 20%

The invention also relates to methods for the production of said yarn.

## Description

The present invention relates to a yarn for the production of fabrics.

The present invention also relates to methods for the production of said yarn.

In detail, the present invention relates to a yarn for the production of loom-woven fabrics and circular woven fabrics or fabrics made using other technologies, characterized by a special combination of elements of a varying nature and by the physical characteristics of the components which, when interacting together, irradiate an energy of considerable force able to influence the equilibrium and dynamics of the energy transmitted and produced by the cells, tissues and body organs and able to improve the quality of life of human being, animal and vegetable world.

The object of the present invention is to provide a yarn which can be used for the manufacture of garments able to provide beneficial physiological effects owing to infrared emissions.

One task of the present invention is to provide a yarn which can be used for the manufacture of garments able to produce heating of the muscles and the articulations. This may be useful, among other things, for providing natural relief to zones affected by muscular pains and/or arthritis.

A further task of the present invention is to provide a yarn by means of which it is possible to obtain garments able to increase the superficial blood circulation in the zones which make contact with the garment, so as to obtain a reduction in the healing time of any inflammations.

The above mentioned object and tasks are achieved by a yarn according to Claim 1 and by a method according to Claim 9.

The technical features and further advantages of the invention will emerge from the description provided hereinbelow by way of a non-limiting example.

At present a yarn which has the technical features of the yarn according to the invention is not present on the market and no other FIR (far infrared ray) radiating material is currently able to achieve the values for FAR energy (infrared rays produced mainly by means of carbon fibres) obtained with the yarn according to the invention.

As is known, solar rays, or in any case infrared rays produced by hot bodies and in particular far infrared rays (FIR), at a wavelength ranging generally between 4 and 14 micrometres (µm), have beneficial and therapeutic properties and scientific studies have shown that they are able to: penetrate the cutaneous and subcutaneous layers; increase the temperature of the body by up to 5 degrees; improve the blood circulation by supplying the tissues with a greater amount of oxygen; increase the metabolism with a greater expulsion of toxins; increase fatigue resistance; as well as increase physical strength and psychic reactivity. According to a modern holistic vision, far infrared rays (FIR) produce beneficial effects of both a physical and a psychical nature in man, whence the importance and significance of this particular type of energy.

All materials present on the Earth, i.e. both inert materials and plant and animal material, may be irradiated by far infrared rays (FIR).

Some materials (such as black bodies), owing to their suitable mineral composition, retain far infrared rays (FIR) in greater amounts than other materials and release them to other materials or living beings which, for the opposite reason, are unable to retain them.

The yarn according to the invention, owing to its particular composition, is able to emit FAR rays radiations, which are generated mainly by means of carbon fibre, in such a large amount that their energy may be immediately perceived by the psycho-physical system of man, triggering all the beneficial effects mentioned above.

On the market there exist other materials which, it is proved, retain and release discrete amounts of far infrared rays (FIR), such as nanoceramics, but the yarn according to the invention, which instead releases FAR rays, is unique and by far more effective than any other material, including nanoceramics.

The yarn consists of carbon, polypropylene, polyamide and microparticles of silver oxide, where "microparticle" is understood as meaning a particle with a diameter of between 1 and 100 nanometres (nm).

The count (weight) of the yarn according to the invention may fall within the range of between 50 Decitex (Dtex) and 300 Decitex (Dtex), where "Decitex" identifies, in a known manner, the weight in grammes of 10,000 metres of yarn.

The yarn according to the invention has the following compositions:
- Polypropylene (PP) in an amount of between 60% and 80%
- Silver oxide (Ag20) in an amount of between 0.5 % and 5%
- Carbon (C60sp2 or Csp2) micronized as a nanopowder in an amount of between 5% and 10%
- Polyamide (PA) in amount of between 10% and 20%

These components assembled together in the percentage amounts depending on their varying count act as receptors of far infrared rays (FIR) and emitters of FAR rays.

The production processes for obtaining the radiating yarn described above may be advantageously as follows:
A) spiralling (winding) a thread of polyamide (PA), coated with a nanopowder of C60sp2 or Csp2 carbon, around the polypropylene (PP) containing the silver oxide (Ag2O).
B) mixing the polypropylene (PP) and polyamide (PA) polymers containing the silver oxide Ag20 and the C60sp2 or Csp2 nanopowder in the percentage amounts described above, extruding them together so as to obtain a yarn with the counts as described above.

In detail, by means of the first method, identified above as method A), a thread comprising polyamide and carbon nanopowder, in the percentage amounts indicated above, is wound around a thread comprising polypropylene and silver oxide, in the percentage amounts indicated above. Thus advantageously a single thread is obtained.

"Carbon nanopowder" is understood as meaning a particulate comprising particles with a size of between 1 nm and 100 nanometres (nm).

By means of the second method described above, which is identified above as method B), firstly a mixture is prepared by mixing together, in the percentage amounts described above, polypropylene (PP) and polyamide (PA) polymers containing the silver oxide (Ag2O) and carbon nanopowder (C60sp2 or Csp2), respectively.

Then, in a known manner, extrusion of the mixture is performed so as to obtain the yarn according to the invention.

Advantageously the polypropylene (PP) and polyamide (PA) polymers containing the silver oxide (Ag2O) and carbon nanopowder (C60sp2 or Csp2) are obtained by mixing chips of polypropylene (PP) together with silver salts and by mixing chips of polyamide (PA) together with micronized carbon powder.

The yarn according to the present invention may be advantageously used for the production of loom-woven fabrics, circular woven fabrics or ladder-proof articles or products made using other technologies or for the production of any other object, including non-textile objects, with a special combination of threads and filaments. Said threads, interacting with each other, absorb a large amount of far infrared rays (FIR) and then irradiate them in a large quantity in the form of positive energy (FAR rays).

In detail, the effectiveness of the yarn thus composed is derived from the physical interaction which occurs between the carbon (generating FAR infrared rays), the silver oxide and the polypropylene which form the yarn according to the invention, without there being any physical interference between the components; the physical interaction increases the power of the radiations of these materials assembled in the correct relative doses, decupling the initial energy.

Tests carried out at a certified laboratory have confirmed the effectiveness of articles of clothing made using percentage amounts of the yarn according to the invention, which may be even relatively small in size, i.e. of the order of 20%.

The tests were carried out on a test group consisting of six male volunteers and were divided into tests performed on a bicycle ergometer and tests performed on a treadmill.

Before starting the tests a blood sample was taken from each tester in order to measure the basic concentration of lactate.

Further samples were then taken during the entire test session in order to assess, under stress conditions, the lactate concentration in the blood.

The tests were carried out using a knitwear garment provided with inserts made with the yarn according to the invention. The inserts had a total surface area of 30 square centimetres (cm²) and were arranged along the sides of the knitwear garment. Wearing of the knitwear garment produced significant variations in the measurements recorded for the entire test group, as follows:
- increase of the heart rate recorded at the anaerobic threshold of 3 bpm (beats per minute), of the average values recorded for the whole test group;
- increase of the power recorded at the anaerobic threshold of 15 Watts, of the average values recorded for the whole test group;
- increase of the maximum power obtained of 15 Watts, of the average values recorded for the whole test group;
- reduction of the lactate concentration in the blood of 3.6 mm/L, of the average values recorded for the whole test group;
- increase of the speed at the anaerobic threshold of 0.5 km/h, of the average values recorded for tests carried out on the treadmill;
- increase of the power/weight ratios recorded at the anaerobic threshold of 0.16 W/kg, of the average values for the entire test group;

"Anaerobic threshold", as is known, is understood as meaning the maximum working load which can be withstood by an athlete for a prolonged period of time, without the accumulation of lactic acid.

The results obtained are summarised in the tables below: The garment provided with inserts made using the yarn according to the invention is indicated as "FAR knitwear", while the garment used as reference garment is indicated as "ref. knitwear".

The tests carried on the ergometer bike are indicated as "Bike", while the treadmill tests are indicated as "treadmill".

**Table 1: Variation in the heart rate at the anaerobic threshold (HB threshold) and variation in the maximum heart beat (HB max)**

| **Heart beat (bpm)** | | | | |
|---|---|---|---|---|
| Tester | HB threshold (ref. knitwear) | HB threshold (FAR knitwear) | HB max (ref. knitwear) | HB max (FAR knitwear) |
| Bike - tester A | 140 | 144 | 160 | 166 |
| Bike - tester B | 161 | 162 | 177 | 172 |
| Bike - tester C | 166 | 167 | 180 | 184 |
| Treadmill - tester D | 179 | 181 | 194 | 196 |
| Treadmill - tester E | 169 | 173 | 185 | 186 |
| Treadmill - tester F | 170 | 172 | 185 | 186 |
| **Average** | 164 | 167 | 180 | 182 |

**Table 2: Variation in lactate concentration in the blood**

| **Lactate (mm/l)** | | |
|---|---|---|
| Tester | Lactate in blood (ref. knitwear) | Lactate in blood (FAR knitwear) |
| Bike - tester A | n/a | 11.8 |
| Bike - tester B | 9.4 | 8.7 |
| Bike - tester C | 15.5 | 8.7 |
| Treadmill - tester D | 5.6 | 2.6 |
| Treadmill - tester E | 5.3 | 3.0 |
| Treadmill - tester F | 14.8 | 9.7 |
| **Average** | 10.1 | 6.5 |

**Table 3: Variation in the power at the anaerobic threshold (P threshold)**

| **Power at the anaerobic threshold (Watt)** | | |
|---|---|---|
| Tester | P threshold (ref. knitwear) | P threshold (FAR knitwear) |
| Bike - tester A | 177 | 187.0 |
| Bike - tester B | 246 | 279.0 |
| Bike - tester C | 235 | 245.0 |
| Treadmill - tester D | 365.7 | 388.5 |
| Treadmill - tester E | 301.8 | 301.8 |
| Treadmill - tester F | 327.3 | 338.6 |
| **Average** | 275 | 290.0 |

**Table 4: Variation in the maximum power obtained (P max)**

| **Maximum power obtained (Watt)** | | |
|---|---|---|
| Tester | P max (ref. knitwear) | P max (FAR knitwear) |
| Bike - tester A | 290 | 301.0 |
| Bike - tester B | 341 | 348.0 |
| Bike - tester C | 320 | 345.0 |
| Treadmill - tester D | 457.1 | 468.5 |
| Treadmill - tester E | 422.8 | 445.7 |
| Treadmill - tester F | 388.5 | 400.0 |
| **Average** | 370 | 385.0 |

**Table 5: Variation in the power/weight ratio at the anaerobic threshold (P/W threshold)**

| **Power/weight ratio (W/kg)** | | |
|---|---|---|
| Tester | P/W threshold (ref. knitwear) | P/W threshold (FAR knitwear) |
| Bike - tester A | 2.68 | 2.83 |
| Bike - tester B | 3.00 | 3.40 |
| Bike - tester C | 3.01 | 3.14 |
| Treadmill - tester D | 3.25 | 3.44 |
| Treadmill - tester E | 3.10 | 3.10 |
| Treadmill - tester F | 2.94 | 3.05 |
| **Average** | 3.00 | 3.16 |

**Table 6: Variation in speed at the anaerobic threshold (V threshold) and variation in the maximum speed (V max)**

| **Speed (km/h)** | | | | |
|---|---|---|---|---|
| Tester | V threshold (ref. knitwear) | V threshold (FAR knitwear) | V max (ref. knitwear) | V max (FAR knitwear) |
| Treadmill - tester D | 16.0 | 17.0 | 20.0 | 20.5 |
| Treadmill - tester E | 15.5 | 15.5 | 18.5 | 19.5 |
| Treadmill - tester F | 14.5 | 15.0 | 17.0 | 17.5 |
| **Average** | 15.33 | 15.83 | 18.50 | 19.17 |

The results above show how the knitwear garment comprising inserts made with the yarn according to the present invention produce an improvement in the performance levels recorded with a significant reduction in the values recorded for lactate concentration in the blood (-35,38%) (see Table 2).

Moreover, knitwear garments with inserts made with the yarn according to the present invention produce a significant increase in both the value for the average power at the anaerobic threshold (see Table 3) and the value for the maximum power obtained (see Table 4).

Moreover, knitwear garments with inserts made with the yarn according to the present invention produce an increase in the anaerobic threshold values both for the heart rate (see Table 1) and for the power produced (see Table 5).

Finally, Table 6 shows how higher values were recorded for the testers both in absolute terms and in vicinity of the anaerobic threshold, with a consequent improvement in the performance achieved.

It should also be noted that these results were obtained with a knitwear garment where the inserts made with the yarn according to the present invention do not exceed 20% of the total yarn of the knitwear garment.

This results in undoubted advantages in economic terms, considering that only a small quantity of fabric produced with the yarn according to the present invention is needed in order to obtain superior results compared to other known fabrics.

Different combinations of the same materials may produce significant results in terms of FAR irradiation; different combinations of the same materials nevertheless fall within the formulation of invention.

With regard to the embodiments of the yarn described the person skilled in the art may, in order to satisfy specific requirements, make modifications to and/or replace elements described with equivalent elements, without thereby departing from the scope of the accompanying claims. The garments made at least partially with the yarn according to the invention may be of any known type and form. For example, such garments may include socks, underwear, knitwear, trousers, tracksuits, etc. The parts of the garment made with the yarn according to the invention will be provided in suitable positions depending on the body zone which is to be directly acted on by the FAR rays. The garments may also be made in bandage form and parts for local application (also elasticized in nature), such as knee bands, arm bands or other bandages arranged over articulations, stomach or back wraps, etc., as will now be clear to the person skilled in the art on the basis of the description provided above. In the case of an insert made with the yarn according to the invention, insertion may be performed both by changing the yarn during weaving and by fixing (by means of stitches or other fixing means) a piece of material made with the yarn according to the invention in a special place or position on the garment manufactured in the traditional manner.

## Claims

1. Yarn for the production of fabrics, suitable for absorbing far infrared rays (FIR) and subsequently irradiating said rays in the form of FAR rays, said yarn being **characterized in that** it is composed of:
- Polypropylene (PP) in an amount of between 60% and 80%
- Silver oxide (Ag20) in an amount of between 0.5 % and 5%.
- Carbon (C60sp2 or Csp2) in an amount of between 5% and 10%
- Polyamide (PA) in amount of between 10% and 20%

2. Yarn according to Claim 1, having a yarn count ranging between 50 Dtex and 300 Dtex.

3. Yarn according to Claim 1, produced with one of the two following processes A and B:
A) winding a thread of polyamide (PA) coated with a carbon nanopowder (C60sp2 or Csp2) around the polypropylene (PP) containing the silver oxide (Ag20) in the polymer matrix;
B) mixing the polypropylene (PP) and polyamide (PA) polymers containing the silver oxide (Ag2O) and the carbon nanopowder (C60sp2 or Csp2) in the percentage amounts described in Claim 1 and extruding them together.

4. Yarn according to Claim 1, wherein the silver oxide (Ag20) is in the form of microparticles with a diameter of between 1 nm and 100 nm.

5. Yarn according to Claim 1, wherein the carbon (C60sp2 or Csp2) is micronized in the form of a powder comprising particles with a size of between 1 nm and 100 nm.

6. Yarn according to Claim 1, wherein a thread comprising polyamide (PA) and carbon nanopowder (C60sp2 or Csp2) is wound around a thread comprising polypropylene (PP) and silver oxide (Ag20).

7. Yarn according to Claim 1, formed by an extrusion of a mixture consisting of polypropylene chips mixed with silver salts and polyamide chips mixed with micronized carbon powder.

8. Garment comprising at least one insert made with the yarn according to any one of Claims 1 to 7.

9. Method for the production of a yarn suitable for absorbing far infrared rays (FIR) and then irradiating said rays in the form of FAR rays, where polypropylene (PP), silver oxide (Ag20), carbon (C60sp2 or Csp2) and polyamide (PA) are combined to form the yarn according to one of the two following processes A) or B):
A) winding a thread of polyamide (PA) coated with carbon nanopowder (C60sp2 or Csp2) around the polypropylene (PP) containing the silver oxide (Ag2O) in the polymer matrix;
B) mixing the polypropylene (PP) and polyamide (PA) polymers containing the silver oxide (Ag2O) and the carbon nanopowder (C60sp2 or Csp2) and extruding the mixture;
and wherein:
- the polypropylene (PP) is in an amount of between 60% and 80%
- the silver oxide (Ag2O) is in an amount of between 0.5 % and 5%.
- the carbon (C60sp2 or Csp2) is in an amount of between 5% and 10%;
- the polyamide (PA) is in amount of between 10% and 20%.

10. Method according to Claim 9, wherein the polypropylene (PP) and polyamide (PA) polymers containing the silver oxide (Ag20) and the carbon nanopowder (C60sp2 or Csp2) are obtained by mixing chips of polypropylene (PP) together with silver salts and by mixing chips of polyamide (PA) together with micronized carbon powder.
